# EUROPEAN PATENT APPLICATION

(11) **EP 1 103 258 A1**
(43) Date of publication of application: **30.05.2001**
(21) Application number: 99931460.2
(22) Date of filing: 21.07.1999
(51) Int. Cl.: A61K 31/19, A61K 31/44, A61K 47/38, A61K 47/34

(54) **SOLID PREPARATION CONTAINING SPARINGLY SOLUBLE NSAIDS**

(30) Priority: 22.07.1998 JP 20632098
(71) Applicant: YAMANOUCHI PHARMACEUTICAL CO. LTD., Tokyo 103-8411 (JP)
(72) Inventor: KISHIMOTO, Hideyuki, Yamanouchi Pharm. Co.,Ltd, Yaizu-shi, Sizuoka 425-0072 (JP); HASHIMOTO, Yoshimi, Yamanouchi Pharm. Co., Ltd, Tokyo 174-8612 12 (JP); TAKAMATSU, Miki, Yamanouchi Pharmaceutical Co. Ltd, Yaizu-shi, Sizuoka 425-0072 (JP); EGAWA, Yasushi, Yamanouchi Pharmaceutical Co., Ltd, Tokyo 174-8612 (JP)
(74) Representative: HOFFMANN - EITLE
(86) International application number: JP9903899
(87) International publication number: WO0004896

(57) **Abstract**

A readily absorbable solid composition comprising sparingly soluble NSAIDs of the propionic acid type, a water-soluble polymeric base, and a nonionic surfactant.

## Description

### Technical Field

The present invention relates to an easily absorbable solid pharmaceutical preparation comprising a soluble propionate-type NSAID, a water-soluble polymer base and a nonionic surfactant.

### Background Art

Among non-steroidal anti-inflammatory drugs (NSAIDs), propionate-type NSAIDs have antiphlogistic, analgesic and antipyretic actions evenly with relatively few side effects, so these are used widely as ingredients in an analgesic, an antipyretic and a remedy for cold. However, the propionate-type NSAIDs include many sparingly soluble chemicals so that when they are used as such, there is a problem with their absorptivity in digestive tracts, particularly with their immediate effect.

Heretofore, various pharmaceutical manufacturing techniques have been examined for the purpose of improving the solubility of sparingly soluble chemicals. For example, JP-A 7-291854 discloses solid dispersions obtained, by grinding sparingly soluble chemicals together with a hydrophilic polymer and a solubility improver in the presence of an aqueous solvent and then removing water therefrom, and describes that these dispersions show behavior different from usual solid dispersions, that is, they are dispersed and dissolved rapidly by forming fine droplets containing the sparingly soluble chemicals in water. Further, JP-A 56-110612 discloses a compression-molded material obtained by blending sparingly soluble chemicals with polyvinyl pyrrolidone etc. (and optionally with a surfactant) and granulating them by fluidized bed granulation, and describes that good results were obtained in an dissolutin test, as compared with compression-molded materials produced by spray drying or wet granulation. JP-A 6-128147 describes that a composition obtained by spraying and adhering a water-soluble polymer solution onto crystalline particles of sparingly water soluble chemicals dispersed in gas and then drying shows excellent dissolution in a disintegration test.

Further, EP274870 discloses capsules comprising a non-steroidal anti-inflammatory drug included in micelles of a surfactant, and describes that the solubility of the drug was improved in a dissolution rate experiment.

However, such an improvement in solubility does not necessarily lead to an improvement in absorption in digestive tracts, resulting often in a failure to improve the absorption.

As the techniques of improving the absorption of sparingly soluble chemicals in digestive tracts, WO96/19239 discloses a solid composition containing sparingly soluble chemicals having been rendered non-crystalline or amorphous, a polymer base and a nonionic surfactant, and describes that administration of the composition into dogs causes an increase in two pharmacokinetic parameters, that is, maximum concentration in plasma (Cmax) and area under a curve of concentration in plasma (AUC), thus achieving an improvement in the absorptivity. However, a reduction in the time for reaching the maximum concentration in plasma (Tmax), which can serve as an indicator of their immediate effect, cannot be achieved.

Meanwhile, Cho et al. have reported that ibuprofen as a sparingly soluble propionate-type NSAID is included in a molar ratio of 2 to 3 in β-cyclodextrin in an attempt at reducing Tmax, but there is the problem that the preparation becomes bulky (Int. J. Pharm., 28, 95-, 1986). Further, JP-A 62-292718 discloses a pharmaceutical preparation having magnesium stearate blended in an amount of 5 to 100 weight % in ibuprofen, and describes that Tmax was reduced. However, this prior method also suffers from the disadvantage that the workability of tablets is deteriorated by mixing a large amount of magnesium stearate etc.

### Disclosure of Invention

The object of the invention is to provide a solid composition particularly having immediate effects with improvements not only in the solubility of sparingly soluble propionate-type NSAIDs but also in the absorptivity thereof in digestive tracts, which can be obtained in a simple manufacturing process requiring no additional productive facilities.

The present inventors made an extensive study for pharmaceutical preparations with improvements in the absorptivity of sparingly soluble NSAIDs, and as a result, they unexpectedly found that unlike general sparklingly soluble chemicals which should be rendered amorphous, sparklingly insoluble propionate-type NSAIDs only show improvements in solubility and absorptivity, particularly in a reduction in Tmax, by simply granulating them together with a water-soluble polymer base and a nonionic surfactant, thus arriving at the invention.

That is, the invention relates to an easily absorbable solid composition comprising sparingly soluble propionate-type NSAIDs, a water-soluble polymer base and a nonionic surfactant. Also, the invention relates to a process for producing an easily absorbable solid composition, which comprises the step of blending a water-soluble polymer base and a nonionic surfactant with sparingly soluble propionate-type NSAIDs. Further, the invention relates to a method of improving the absorptivity of sparingly soluble propionate-type NSAIDs, which comprises blending a water-soluble polymer base and a nonionic surfactant with sparingly soluble propionate-type NSAIDs.

The solid composition of the invention is superior to the prior art in that (1) it is not necessary to render chemicals amorphous, (2) the complicated process of e.g. mixing and grinding chemicals by adding an aqueous solvent is not necessary, (3) additional productive facilities are not necessary because the process is not limited by a special step of granulation, such as fluidized bed granulation, spray drying, etc., (4) there are none of such problems as bulkiness and deterioration in workability, and (5) its immediate effect can be expected because of a reduction in Tmax.

Hereinafter, the present invention is described in more detail.

The sparingly soluble propionate-type NSAIDs of the invention includes ibuprofen, phenoprofen, ketoprofen, pranoprofen, naproxen and flurubiprofen. The compound achieving the most significant improvements in solubility and absorptivity by the present invention is ibuprofen.

The water-soluble polymer base used in the invention includes hydroxypropyl methyl cellulose (abbreviated hereinafter to HPMC), hydroxypropyl cellulose (abbreviated hereafter to HPC), polyvinyl pyrrolidone (abbreviated hereinafter to PVP), methyl cellulose (abbreviated hereinafter to MC), ethyl cellulose (abbreviated hereinafter to EC), macrogal, hydroxyethyl cellulose etc. Among these, HPMC, HPC, PVP and MC are particularly preferable.

The amount of the water-soluble polymer base blended is from 0.01 part to 10 parts by weight, preferably from 0.05 part to 5 parts by weight, more preferably from 0.05 part to 1 part by weight, relative to 1 part by weight of ibuprofen. This is because in an amount of less than 0.01 part by weight, the absorption of chemicals cannot be improved in many cases. On the other hand, if the amount is more than 10 parts by weight, the resulting pharmaceutical preparation becomes bulky and difficult to administer, thus making it practically undesirable. One or more water-soluble polymer bases can be used as necessary.

The nonionic surfactant used in the present invention includes sucrose ester of fatty acid (sugar esters), polyoxyethylene hydrogenated castor oil, polyoxyethylene sorbitan fatty esters, block polymer-type ethers etc. Among these, polyoxyethylene hydrogenated castor oil is particularly preferable.

The polyoxyethylene hydrogenated castor oil includes e.g. hydrogenated castor oil polyoxyethylene ethers, polyoxyethylene hydrogenated castor oil, polyoxyethylene hydrogenated castor oil (20E.O), polyoxyethylene hydrogenated castor oil (5E.O), polyoxyethylene hydrogenated castor oil 50 (HCO-50), polyoxyethylene hydrogenated castor oil 60 (HCO-60), etc.

The polyoxyethylene sorbitan fatty esters include e.g. Polysorbate 40 (Tween 40), Polysorbate 60 (Tween 60), Polysorbate 65, Polysorbate 80 (Tween 80) and monolaurate polyoxyethylene sorbitan (20E.O).

The block copolymer-type ethers include e.g. polyoxyethylene [160] polyoxypropylene [30] glycol (Pluronic F68) and polyoxyethylene oxypropylene cetyl ether (20E.O 4P.O).

The amount of the nonionic surfactant blended is from 0.01 part to 5 parts by weight, preferably from 0.05 part to 2 parts by weight, more preferably from 0.05 to 1 part by weight, relative to 1 part by weight of ibuprofen. This is because in an amount of less than 0.01 part by weight, the absorption of chemicals cannot be improved in many cases, while an amount of more than 5 parts by weight is practically not preferable because production of the pharmaceutical preparation may be difficult depending on the type of the nonionic surfactant. One or more nonionic surfactants can be used as necessary

The ratio by weight of ibuprofen to the water-soluble polymer base and the nonionic surfactant used in the invention is selected such that ibuprofen : water-soluble polymer base : nonionic surfactant is 1 : (0.01 to 10) : (0.01 to 5). This ratio is preferably 1 : (0.05 to 5) : (0.05 to 2), more preferably 1 : (0.05 to 1) : (0.05 to 1).

The solid composition of the invention is obtained by adding a binder to sparingly soluble propionate-type NSAIDs, the water-soluble polymer base and the nonionic surfactant and then granulating the mixture, or by adding the nonionic, surfactant dissolved in a binder to sparingly soluble propionate-type NSAIDs and the water-soluble polymer base and then granulating the mixture, or by adding pharmaceutically acceptable fillers to such a mixture, then granulating the mixture and removing the solvent as necessary by vacuum drying, air drying, fluidized-bed drying etc. Alternatively, the solid composition can be obtained by spray-drying or freeze-drying a solution or dispersion of the ingredients. The granulation method is not limited to the examples described above.

The resulting granules may be used as such or may be formulated by pharmaceutically acceptable fillers into solid pharmaceutical preparations in generally known forms such as powder, finely divided particles, granules, tablets, capsules, chewable tablets, effervescent, pills, tablets dissolved just before administration, granules dissolved just before administration, finely divided particles dissolved just before for administration, etc.

In the invention, the mere "granulation" refers to every procedure for producing granules (i.e. particles) of almost uniform shape and size from the starting pharmaceutical materials in various forms of powder, bulk mass, solution or melt, and the "granulation" by a specific method means that granules of almost uniform shape and size are produced by the specific method.

Specifically, there are the following methods: (1) an extrusion granulation method wherein a binder solution is added to and kneaded with the starting powder, and the material thus kneaded and combined is molded and extruded by pressing the material against dies or a screen and then extruding it therethrough in a cylindrical granulator or the like, (2) a grinding granulation method of grinding a wet intimate mixture into particles of a predetermined size by a speed mill (Showa Engineering Co., Ltd.), a power mill (Dalton Co., Ltd.) or the like, (3) a stirring granulation method wherein a binder solution is added to the starting powder which is then granulated under mixing and stirring by a Henshel mixer (Rheinstahl Henshel AG), a planetary mixer or the like, (4) a rolling granulation method wherein the starting material previously rolled by a centrifugal fluidizing granulator (produced by e.g. Freund Sangyo Co., Ltd.), a rotoprocessor (Eromatic Fuji Sangyo Co., Ltd.) or a Marumerizer (Fuji Powdal Co., Ltd.) is sprayed or coated with a binder to produce spherical particles, (5) a spray drying method using a spray drying machine (produced by e.g. Ohkawara Kakoki Co., Ltd.) by which droplets spayed with a liquid or a suspension are dried, and (6) a fluidized-bed granulation method using a fluidized-bed granulator (e.g. Spiral Flow produced by Freund Sangyo Co., Ltd. and New Marumerizer produced by Fuji Powdal Co., Ltd.) in which a fluidized bed of the starting powder is formed with an air stream, then dried and simultaneously sprayed with a binder solution, whereby the particles are granulated by adhering and aggregating them via liquid cross-linking.

The solid composition of the invention can be used for any purposes and in any amounts within a generally conceivable range of sparingly soluble proplonate-type NSAIDs, and for example, it can be used directly as an antipyretic analgesic. Further, pharmaceutically acceptable ingredients can also be incorporated into it. The ingredients which can be incorporated include other antipyretic and analgesic ingredients, narcotic and analgesic ingredients, caffeine and analogues thereof, vitamins, gastric antacids, crude drugs etc.

The other antipyretic and analgesic ingredients include e.g. aspirin, ethenzamide, sasapyrine, salicylamide, sodium salicylate, isopropyl antipyrine, loxoprofen sodium, diclofenac sodium, piroxicam etc. The narcotic and analgesic ingredients include e.g. bromvalerylurea, allyl isopropyl acetyl urea etc. Caffeine and analogues thereof include e.g. caffeine, anhydrous caffeine, sodium caffeine benzoate, etc. The vitamins include e.g. vitamin B₁, vitamin B₂ and vitamin C as well as derivatives thereof and salts thereof. The gastric antacids include e.g. magnesium silicate, synthetic aluminum silicate, synthetic hydrotalcite, co-precipitates of aluminum hydroxide/sodium hydrogen carbonate, dried gel of aluminum hydroxide, magnesium metasilicate aluminate, amino acetic acid, etc. The crude drugs include e.g. licorice, cinnamon, peony, JIRYU, zanthoxylum, ginger, dried orange peel, etc.

Further, the solid composition of the invention can also be used as a remedy for cold by blending various pharmaceutically acceptable ingredients. The ingredients capable of being blended include e.g. other antipyretic and analgesic ingredients, anti-histamine ingredients/anti-allergic ingredients, coughing-relieving ingredients, expcetorative ingredients, bronchus-expanding ingredients, herb medicines, caffeine or analogues thereof, vitamins, gastric antacids, crude drugs etc.

The anti-histamine ingredients/anti-allergic ingredients include e.g. chlorphenylamine maleate, diphenhydramine hydrochloride, diphenylpyraline hydrochloride, alimemazine tartrate, clemastine fumarate, carbinoxamine maleate, cyproheptadine hydrochloride, mequitazine, ketotifen fumarate, azelastine hydrochloride, Astemizole, ebastine, tranilast, emedastine difumarate, oxatomide, etc.

The coughing relieving ingredients include e.g. dextromethorphan hydrobromide, dimemorfen phosphate, codeine phosphate, dihydrocodeine phosphate, tipepidine citrate, tipepidine hibenzate, AROCRAMIDE hydrochloride, noscapine, noscapine hydrochloride, etc.

The expcetorative ingredients include e.g. potassium guaiacol sulfonate, guaifenesin, lysozyme chloride, ethylcysteine hydrochloride, methylcysteine hydrochloride, L-carbocysteine, ambroxol hydrochloride, BROMHEXINE hydrochloride, acetyl cysteine, etc.

The bronchus-expanding ingredients include e.g. methyl ephedrine hydrochloride, methyl ephedrine saccharine salt, phenyl propanol amine, formoterol fumarate, procatechol hydrochloride, isoprenaline hydrochloride, flutropium bromide, beclometasone propionate, isoprenaline sulfate, salbutamol sulfate, trimetoquinol hydrochloride, etc.

The herb medicines include e.g. KAKKONTO, KEISHITO, KOASAN, saikokeishito, syosaikoto, syoseiryuto, BAKUMONTOTO, hangekobokuto, MAOTO, etc.

The above-mentioned ingredients can be blended as the other antipyretic and analgesic ingredients, caffeine or analogues thereof, vitamins, gastric antacids, crude drugs etc.

### Brief Description of the Drawing

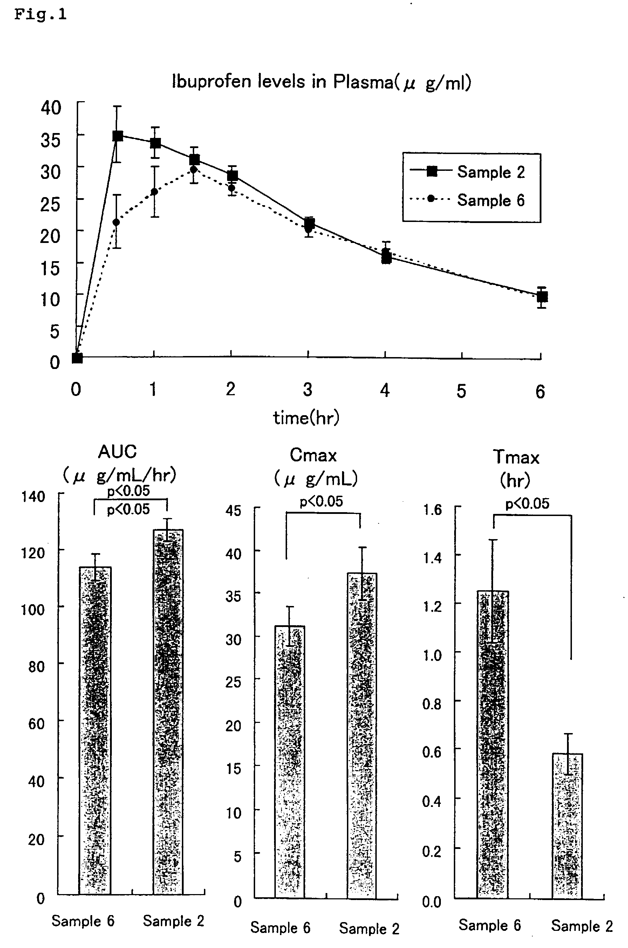

Fig. 1 shows ibuprofen levels in plasma and pharmacokinetic parameters upon oral administration of Samples 2 and 6 to dogs.

### Best Mode for Carrying Out the Invention

Hereinafter, the invention is described in detail by reference to the Examples and Test Examples.

### Example 1

10 parts by weight of ibuprofen, 2 parts by weight of HPMC, 2 parts by weight of macrogal and 2 parts by weight of polyoxyethylene hydrogenated castor oil (HCO-60) were taken, then 2 parts by weight of ethanol was added thereto, and the mixture was kneaded in a mortar. The ethanol was evaporated to give granules. These were designated Sample 1.

### Example 2

10 parts by weight of ibuprofen, 2 parts by weight of HPMC and 2 parts by weight of polyoxyethylene hydrogenated castor oil (HCO-60) were taken, then 2 parts by weight of ethanol was added thereto, and the mixture was kneaded in a mortar. The ethanol was evaporated to give granules. Tablets each containing 100 mg of ibuprofen and consisting of 75 parts by weight of these granules, 20 parts by weight of fine crystalline cellulose and 5 pats by weight of partially pregelatinized starch were produced in a usual manner and designated Sample 2.

### Example 3

20 parts by weight of ibuprofen, 12 parts by weight of HPC and 1 part by weight of polyoxyethylene hydrogenated castor oil (HCO-60) were taken, then 8 parts by weight of ethanol was added thereto and the mixture was kneaded in a mortar. The ethanol was evaporated to give granules.

### Example 4

20 parts by weight of ibuprofen and 1 part by weight of PVP were placed in a kneader, 4 parts by weight of polyoxyethylene sorbitan fatty ester (Tween 80) dissolved in 5 parts by weight of a mixture of ethanol and water (2 : 1) was added thereto, and the mixture was kneaded. The solvent was evaporated to give granules. Further, these were encapsulated to give capsules.

### Example 5

10 parts by weight of ibuprofen, 6 parts by weight of hydroxyethyl cellulose and 2 parts by weight of polyoxyethylene [160] polyoxypropylene [30] glycol (Pluronic F68) were taken, then 4 parts by weight of methanol was added thereto, and the mixture was kneaded in a planetary mixer. The methanol was evaporated to give granules. By regulating the size of these granules by a power mill, finely divided particles were obtained.

### Example 6

10 parts by weight of ibuprofen and 2 parts by weight of HPMC were placed in a high-speed stirring granulator, then 2 parts by weight of polyoxyethylene hydrogenated castor oil (HCO-60) dissolved in 3 parts by weight of a mixture of ethanol : water (1 : 1) was added thereto, and the mixture was kneaded. 10 parts of fine crystalline cellulose was added thereto and the mixture was further kneaded. The solvent in this kneaded material was evaporated to give granules. Tablets each containing 75 mg of ibuprofen and consisting of 85 parts by weight of these granules, 10 parts by weight of fine crystalline cellulose and 5 pats by weight of partially pregelatinized starch were produced in a usual manner and designated Sample 3.

### Example 7

10 parts by weight of ibuprofen and 2 parts by weight of HPMC were placed in a fluidized-bed granulator and granulated with 2 parts by weight of polyoxyethylene hydrogenated castor oil (HCO-60) dissolved in 3 parts by weight of a mixture of water: ethanol (1 : 1). Tablets each containing 75 mg of ibuprofen and consisting of 75 parts by weight of these granules, 20 parts by weight of fine crystalline cellulose and 5 pats by weight of partially pregelatinized starch were produced in a usual manner and designated Sample 4.

### Example 8

HPMC and polyoxyethylene hydrogenated castor oil (HCO-60) in the amounts shown in Table 1 were mixed with 10 parts by weight of ibuprofen and dissolved in dichloromethane. These solutions were spray-dried by a spray drying machine to give Samples 5-1 to 5-7.

**Table 1**

| Samples | 5-1 | 5-2 | 5-3 | 5-4 | 5-5 | 5-6 | 5-7 |
|---|---|---|---|---|---|---|---|
| HPMC | 2 | 4 | 5 | 10 | 15 | 20 | 30 |
| HCO-60 | 2 | 2 | 5 | 5 | 5 | 5 | 5 |
| (unit: parts by weight) | | | | | | | |

### Comparative Example

A commercial pharmaceutical preparation containing 100 mg ibuprofen. This was designated Sample 6.

### Test Example 1

Each sample was tested by the puddle method in an dissolution test according to the Japanese Pharmacopoeia, and the amount of ibuprofen (µg/ml) dissolved for 5 minutes in the test was determined. Water was used as the test solution, the amount of the dissolution medium was 500 ml, and the number of puddle revolutions was 100 rpm. The raw ibuprofen material, Samples 5-1 to 5-7, and Sample 6 were examined respectively in the test. The content of ibuprofen in every sample was 150 mg. The results are shown in Table 2.

**Table 2**

| Samples | Raw material | 1 | 5-1 | 5-2 | 5-3 | 5-4 | 5-5 | 5-6 | 5-7 | 6 |
|---|---|---|---|---|---|---|---|---|---|---|
| Dissolved amount | 5.7 | 62.8 | 55.2 | 61.8 | 54.8 | 78.8 | 73.0 | 75.4 | 97.6 | 0.0 |
| (unit: µg/ml) | | | | | | | | | | |

The granules produced according to the invention were proven to show good dissolution as compared with the amounts dissolved from the raw material and Sample 6 (Comparative Example). Further, solubility was shown to be improved similarly by simple kneading in a mortar as used for Sample 1 or by spray-drying as used for Samples 5-1 to 5-7.

### Test Example 2

Each sample was tested by the puddle method in an dissolution test according to the Japanese Pharmacopoeia, and the amount of ibuprofen (µg/ml) dissolved for 5 minutes in the test was determined. Water was used as the test solution, the amount of the dissolution medium was 500 ml, and the number of puddle revolutions was 100 rpm. Samples 3, 4 and 6 were examined respectively in the test. The results are shown in Table 3.

**Table 3**

| Samples | 3 | 4 | 6 |
|---|---|---|---|
| Dissolved amount | 42.5 | 47.5 | 0.0 |
| (unit: µg/ml)) | | | |

The tablets produced according to the invention were proven to show good dissolution as compared with the amount dissolved from Sample 6 (Comparative Example). Further, the dissolved amount was the same regardless of whether the granules were produced by stirring granulation (Sample 3) or fluidized-bed granulation (Sample 4). From this result along with the result in Test Example 1, it was found that the present invention can be practiced regardless of the granulation method.

### Test Example 3

Five male beagle dogs each weighing about 10 kg were fasted for 18 hours from the day before administration and orally given Sample 2 or 6 together with about 20 ml water. Blood was collected from foreleg veins at predetermined intervals by an injection tube treated with heparin. The collected blood was centrifuged at 3000 rpm for 15 minutes to give plasma. The amount of ibuprofen in this plasma was measured by high performance liquid chromatography. The results are shown in Fig. 1.

The amount of ibuprofen in each sample (Sample 2 and Sample 6 (Comparative Example 1)) administered was 100 mg.

As shown in Fig. 1, Sample 2 shows considerable improvements in AUC and Cmax and a significant reduction in Tmax as compared with those of Sample 6. From this result, it was confirmed that the solid composition of the present invention can be expected to have an immediate effect with an improvement in the absorptivity of ibuprofen.

### Example 9

1 part by weight of a water-soluble polymer base and 1 part of a nonionic surfactant shown in Table 4 were mixed with 5 parts by weight of ibuprofen, and after ethanol was added thereto, the mixture was kneaded in a mortar, and the solvent was evaporated by an air dryer to give granules. These were designated Samples 7-1 to 7-9., However, because Sample 7-4 was a paste after drying, 6 parts by weight of crystalline cellulose was added thereto as a powdering agent, and the mixture was kneaded and dried in the same manner as above to give a granulated sample.

### Test Example 4

Samples 7-1 and 7-9, and the raw ibuprofen powder as the control, were examined by the puddle method in an dissolution test according to the Japanese Pharmacopoeia, and the amount of ibuprofen (µg/ml) dissolved for 5 minutes in the test was determined. The test solution used was a first solution (pH 1.2) in a disintegration test in the Japanese Pharmacopoeia, the amount of the dissolutuion medium was 500 ml, and the number of puddle revolutions was 50 rpm. The content of ibuprofen in every sample was 150 mg. The results are shown in Table 4.

**Table 4**

| Sample | Water-soluble polymer base | Nonionic surfactant | Dissolved amount |
|---|---|---|---|
| Raw material | - | - | 0.00 |
| 7-1 | HPMC | HCO-60 | 67.73 |
| 7-2 | HPC | HCO-60 | 80.04 |
| 7-3 | MC | HCO-60 | 67.73 |
| 7-4 | PVP | HCO-60 | 151.88 |
| 7-5 | macrogal | HCO-60 | 20.52 |
| 7-6 | EC | HCO-60 | 22.58 |
| 7-7 | HPMC | Tween 80 | 98.52 |
| 7-8 | HPMC | Pluronic F68 | 51.31 |
| 7-9 | HPMC | Sucrose fatty ester | 18.47 |
| (unit: µg/ml)) | | | |

The raw ibuprofen material was not dissolved in the first solution (pH 1.2) in the disintegration test in the Japanese Pharmacopoeia, but the granules produced according to the present invention were proven to have improved solubility regardless of the types of water-soluble polymer base and nonionic surfactant.

### Example 10

1 part by weight of HPMC and 1 part by weight of polyoxyethylene hydrogenated castor oil (HCO-60) were mixed with 5 parts by weight of chemicals shown in Table 5, and after ethanol was added thereto, each mixture was kneaded in a mortar, and the solvent was evaporated by an air dryer to give granules. These were designated Samples 8-1 to 8-9.

### Test Example 5

Samples 8-1 and 8-9, and raw materials of various chemicals as the control, were examined by the puddle method in an dissolution test according to the Japanese Pharmacopoeia, and the amount of ibuprofen (µg/ml) dissolved for 5 minutes in the test was determined. The test solution used was the first solution (pH 1.2) in a disintegration test in the Japanese Pharmacopoeia, the amount of the dissolution medium was 500 ml, and the number of puddle revolutions was 50 rpm. Each sample contains a dose of each chemical. The results are shown in Table 5.

**Table 5**

| Sample | Chemical | Dissolved amount | |
|---|---|---|---|
| | | Sample | Control |
| 8-1 | Ibuprofen | 67.73 | 0.00 |
| 8-2 | Phenoprofen | 912.60 | 11.47 |
| 8-3 | Ketoprofen | 10.42 | 1.57 |
| 8-4 | Pranoprofen | 90.19 | 17.81 |
| 8-5 | Naproxen | 40.52 | 5.92 |
| 8-6 | Flurbiprofen | 8.26 | 0.22 |
| 8-7 | Indomethacin | 0.78 | 0.09 |
| 8-8 | Loxoprofen | 121.64 | 101.73 |
| 8-9 | Nifedipine | 0.00 | 0.00 |
| (unit: µg/ml)) | | | |

Samples 8-1 to 8-6 whose chemicals are sparingly soluble propionate-type NSAIDs have significantly improved solubility as compared with their raw materials. Samples 8-7 whose chemical is a sparingly soluble aryl acetate-type NSAID has improved solubility, but it is still poor insolubility and somewhat inferior in practical use to the sparingly soluble propionate-type NSAIDs. On the other hand. Sample 8-8 whose chemical is a non-sparingly-soluble propionate-type NSAID does not show further improvements in solubility, and Sample 8-9 whose chemical is a sparingly soluble hypotensive drug nifedipine does not show any improvement in solubility. From the results described above, it was revealed that the pharmaceutical manufacturing techniques of the present invention are effective for only the sparingly soluble propionate-type NSAIDs in particular among sparingly soluble chemicals, and are effective for only those sparingly soluble among propionate-type NSAIDs.

### Example 11

10 parts by weight of ibuprofen and 3 parts by weight of HPC were placed in a planetary mixer, then a solution of 2 parts by weight of polyoxyethylene [160] polyoxypropylene [30] glycol (Pluronic F68) in 3 parts by weight of a mixture of ethanol and water (1 : 1) was added thereto and the mixture was kneaded. 10 parts by weight of fine crystalline cellulose was added thereto and the mixture-was further kneaded. The solvent in this kneaded material was evaporated to give granules. Separately, 60 parts by weight of dimemorfan phosphate, 7 parts by weight of d-chlorpheniramine maleate, 120 parts by weight of dl-methyl ephedrine hydrochloride, 150 parts by weight of anhydrous caffeine, 100 parts by weight of lactose and 53 parts by weight of corn starch were formed in the same manner into granules using 100 parts by weight of 10 % aqueous PVP solution as a binder. Tablets each containing 75 mg ibuprofen and consisting of 75 parts by weight of ibuprofen granules, 50 parts by weight of dimemorfan granules, 13 parts by weight of fine crystalline cellulose and 7 pats by weight of partially pregelatinized starch were obtained in a usual manner.

### Example 12

10 parts by weight of ibuprofen and 2 parts by weight of HPMC were placed in a high-speed stirring granulator, then a solution of 2 parts by weight of polyoxyethylene hydrogenated castor oil (HCO-60) in 3 parts by weight of a mixture of ethanol and water (1 : 1) was added thereto, and the mixture was kneaded. 10 parts by weight of fine crystalline cellulose was added thereto and the mixture was further kneaded. The solvent in this kneaded material was evaporated to give granules. Separately, 6 parts by weight of dihydrocodeine phosphate, 2 parts by weight of chlorpheniramine maleate, 16 parts by weight of dl-methyl ephedrine hydrochloride, 20 parts by weight of anhydrous caffeine, 60 parts by weight of lactose and 20 parts by weight of corn starch were formed in the same manner into granules using 30 parts by weight of 10 % aqueous HPC solution as a binder. Tablets each containing 50 mg ibuprofen and consisting of 36 parts by weight of ibuprofen granules, 51 parts by weight of dihydrocodeine granules, 11 parts by weight of fine crystalline cellulose and 5 pats by weight of partially pregelatinized starch were obtained in a usual manner.

### Industrial Applicability

According to the present invention, there can be provided a solid composition particularly having immediate effects with improvements not only in the solubility of sparingly soluble propionate-type NSAIDs but also in the absorptivity thereof in digestive tracts, which can be obtained in a simple manufacturing process requiring no additional productive facilities

## Claims

1. An easily absorbable solid composition comprising sparingly soluble propionate-type NSAIDs, a water-soluble polymer base and a nonionic surfactant.

2. The solid composition according to claim 1, wherein the sparingly soluble propionate-type NSAIDs are one or more members selected from ibuprofen, phenoprofen, ketoprofen, pranoprofen, naproxen, and flurbiprofen.

3. The solid composition according to claim 1, comprising 1 part by weight of sparingly soluble propionate-type NSAIDs, 0.01 part to 10 parts by weight of a water-soluble polymer base and 0.01 part to 5 parts by weight of a nonionic surfactant.

4. The solid composition according to claim 1, wherein the sparingly soluble propionate-type NSAIDs are ibuprofen.

5. The solid composition according to claim 1, wherein the water-insoluble polymer base is one or more members selected from hydroxypropyl methyl cellulose, hydroxypropyl cellulose, polyvinyl pyrrolidone, methyl cellulose, ethyl cellulose and macrogal.

6. The solid composition according to claim 1, wherein the nonionic surfactant is one or more members selected from polyoxyethylene hydrogenated castor oil, polyoxyethylene sorbitan fatty esters, block polymer-type ethers and sucrose ester of fatty acid.

7. The solid composition according to claim 1, comprising ibuprofen, hydroxypropyl methyl cellulose and polyoxyethylene hydrogenated castor oil.

8. A method of improving the absorptivity of sparingly soluble propionate-type NSAIDs, which comprises blending a water-soluble polymer base and a nonionic surfactant with sparingly soluble propionate-type NSAIDs.
